# EUROPÄISCHE PATENTSCHRIFT

(11) **EP 1 000 922 B1**
(45) Veröffentlichungstag und Bekanntmachung des Hinweises auf die Patenterteilung: **18.09.2002**
(21) Anmeldenummer: 99122636.6
(22) Anmeldetag: 13.11.1999
(51) Int. Cl.: C07C 45/67, C07C 49/203

(54) **Verfahren zur Herstellung von höheren ungesättigten Ketonen**
Process for the preparation of higher unsaturated ketones
Procédé pour la préparation de cétones supérieures non saturées

(30) Priorität: 16.11.1998 DE 19852691
(43) Veröffentlichungstag der Anmeldung: 17.05.2000
(73) Patentinhaber: BASF AKTIENGESELLSCHAFT, 67056 Ludwigshafen (DE)
(72) Erfinder: Oost, Carsten, Dr., 67098 Bad Dürkheim (DE); Stroezel, Manfred, 68549 Ilvesheim (DE); Etzrodt, Heinz, Dr., 67434 Neustadt (DE); Weller, Dietmar, Dr., 67067 Ludwigshafen (DE); Rheude, Udo, Dr., 67166 Otterstadt (DE); Kaibel, Gerd, Dr., 68623 Lampertheim (DE); Krug, Thomas, 67549 Worms (DE); Spiske, Luise, Dr., 64342 Seeheim-Jugenheim (DE); Jaedicke, Hagen, Dr., 67069 Ludwigshafen (DE)

(56) Entgegenhaltungen:
- EP-A- 0 022 955
- DE-B- 1 073 476
- FR-A- 2 371 411
- CHEMICAL ABSTRACTS, vol. 102, no. 25, 24. Juni 1985 (1985-06-24) Columbus, Ohio, US; abstract no. 220448, GESE J ET AL: "Unsaturated ketones" XP002131761 & CS 216 360 B (CZECH.)

## Beschreibung

Die vorliegende Erfindung betrifft ein verbessertes Verfahren zur Herstellung von höheren ungesättigten Ketonen durch Umsetzen der entsprechenden α,β-ungesättigten Alkohole mit Acetessigsäurealkylestern in einer Carroll-Reaktion.

Diese Reaktion ist, sieht man von den erfindungsgemäßen Verbesserungen ab, in ihren wesentlichen Merkmalen bereits bekannt. Erstmals wurde eine derartige Umsetzung zwischen einem ungesättigten Alkohol und einem Acetessigsäurealkylester von Carroll in J. Chem. Soc. (London), 1940, Seiten 704 bis 706, beschrieben. Über den Anwendungsbereich und den Mechanismus dieser Reaktion wurde ein Jahr später von demselben Autor in J. Chem. Soc. (London), 1941, Seiten 507 bis 511, berichtet.

Eine Verfahrensvorschrift für die Herstellung von 6,10,14-Trimethyl-5-pentadecen-2-on durch Umesterung von Acetessigsäureethylester mit 3,7,11-Trimethyl-1-dodecen-3-ol in Anwesenheit von Aluminiumtrialkoholaten ist der französchen Patentschrift 1 219 166 (von 1959) zu entnehmen. Gemäß diesem Verfahren werden die Reaktanten und der Katalysator gemeinsam in der Reaktionsblase vorgelegt und die Reaktion unter destillativer Abtrennung des aus dem Acetessigsäureethylester freiwerdenden Alkohols diskontinuierlich durchgeführt. Dabei wird das gewünschte Keton in einer Reaktionszeit von etwa 10 Stunden in 77%iger Ausbeute erhalten.

Für eine technische Synthese unbefriedigend an diesem Verfahren sind sowohl die verhältnismäßig langen Reaktionszeiten als auch die unzureichenden Ausbeuten. Die unzureichenden Ausbeuten sind besonders gravierend bei der Herstellung von höheren Ketonen, d.h. bei Verwendung von höheren Alkoholen der Formel II, da diese mit wachsender Kettenlänge zunehmend teurer in ihrer Herstellung werden. Versucht man die Ausbeuten dadurch zu verbessern, daß man die preiswertere Komponente, hier den Acetessigsäurealkylester, im Überschuß verwendet, so kommt es leicht zur Bildung von Dehydrazetsäure als Nebenprodukt, die einerseits den Katalysator desaktiviert und andererseits aus dem gewünschten Produkt nur schwer wieder abzutrennen ist. Zudem kann die Dehydrazetsäure auskristallisieren und dadurch die Ablaufleitungen der verwendeten Kolonnen verstopfen.

Es ist eine Reihe weiterer Patentschriften bekannt, in denen verschiedene Varianten der sogenannten Carroll'schen Reaktion beschrieben werden. So heißt es in US-2 795 617 (von 1957) bzw. DE AS 1 053 498 (von 1959) bzw. CH- 342 947 (von 1959), daß "obschon es in der Regel weder notwendig noch erwünscht sei, ein Lösungsmittel verwendet werden kann, um den exothermen Reaktionsverlauf zu mildern". Gemäß den Verfahren dieser Patentschriften wurde das Aluminiumtrialkoholat zu dem Acetoacetat des α,β-ungesättigten Alkohols zugegeben und die Mischung unter starkem Rühren und unter Rückfluß zum Sieden erhitzt. Hierbei wurden Ausbeuten bis zu 80 % der Theorie erreicht. Nachteilig an diesem Verfahren ist, daß die Herstellung des als Ausgangsverbindung verwendeten Acetoacetats in einer vorangehenden Stufe erfolgen muß.

In US 2 839 579 (von 1958) bzw. DE 1 078 112 (von 1960) wird berichtet, daß die Umsetzung in einem Lösungsmittel durchgeführt werden kann. Die Herstellung des entsprechenden Acetoacetats erfolgt durch Kondensation des entsprechenden ungesättigten Alkohols mit Diketen in einer separaten Stufe.

Auch in DE 1 068 696 heißt es, daß die Mitverwendung eines Lösungsmittels vorteilhaft sein könnte. In allen Fällen werden hochsiedende Lösungsmittel genannt, deren Siedepunkte weit oberhalb der Reaktionstemperatur liegen.

Nachteilig an diesem Verfahren ist, daß die in diesen Patenten angegebenen Ausbeuten für eine technische Anwendung unbefriedigend sind und insbesondere, daß zur Herstellung des Acetoacetats des α,β-ungesättigten Alkohols eine zusätzliche Verfahrensstufe erforderlich ist, was zu zusätzlichen Kosten führt. Auch die vorgeschlagene Mitverwendung eines hochsiedenden Lösungsmittels bringt im allgemeinen keine nennenswerten Ausbeutesteigerungen mit sich und führt daher nur zu einer Reduzierung der Raumzeitausbeute.

In dem Tschechischen Patent 216 360 (von 1979) wird empfohlen, Carroll-Reaktionen in einem Gemisch aus dem als Reaktionsprodukt zu erwartenden ungesättigten Keton und dem Acetessigsäuremethylester bzw. -ethylester unter Zugabe einer gerade zur Aufrechterhaltung der Reaktion erforderlichen Menge des ungesättigten Alkohols durchzuführen. Dabei wird aus dem Reaktionsgemisch das Kohlendioxid und ein Gemisch aus dem nicht umgesetzten ungesättigten Alkohol und Methanol bzw. Ethanol abdestilliert, welches kontinuierlich in einer angekoppelten Destillationskolonne fraktioniert wird. Der α,β-ungesättigte Alkohol, dessen Siedepunkt unter 180°C liegen muß, wird anschließend in die Reaktion zurückgeführt. Bei diesem Verfahren wurden bei Reaktionszeiten von 8 Stunden Ausbeuten von ca. 80 % der Theorie erreicht. Nachteilig an diesem Verfahren ist, daß durch die zusätzliche Destillationskolonne zusätzliche Investitions- und Energiekosten entstehen. Zudem sind die Ausbeuten und Reaktionszeiten bei diesem Verfahren für ein modernes technisches Verfahren unbefriedigend.

Weiterhin ist aus DE 2 928 944 (von 1979) die Herstellung von α,β-ungesättigten Ketonen durch Carroll-Reaktion unter Mitverwendung von kleinen Mengen eines Lösungsmittels beschrieben, dessen Siedepunkt zwischen dem des eingesetzten Acetessigsäurealkylesters und dem des hieraus abzuspaltenden Alkohols liegt. Dieses Lösungsmittel wird hierin als "Zwischensieder" bezeichnet. Als mögliche inerte Zwischensieder werden entsprechend siedende Alkohole, Ester, Ether, halogenierte Kohlenwasserstoffe und aromatische Kohlenwasserstoffe ,vorzugsweise aliphatische Ketone mit 4 bis 7 C-Atomen genannt. Als besonders vorteilhafte Ausführungsform wird die Verwendung von 2-Methyl-3-buten-2-ol als reaktivem Zwischensieder genannt, wobei als zusätzliche erwünschte Nebenreaktion dessen Umsetzung mit dem Acetessigsäurealkylester zu 2-Methyl-2-hepten-6-on als weiterem Wertprodukt stattfindet. Als Vorteile der Verwendung eines solchen Zwischensieders werden erhöhte Produktausbeuten (ca. 95 % der Theorie, bezogen auf den Alkohol, und ca. 85 % der Theorie, bezogen auf den Acetessigester, sowie kürzere Reaktionszeiten (ca. 4-5 h) und damit hohe Raumzeitausbeuten genannt. In allen Ausführungsbeispielen werden Reaktionstemperaturen von maximal 165°C angewendet.

Die Verwendung eines Zwischensieders bringt jedoch nicht nur Vorteile mit sich, sondern auch die folgenden Nachteile. So verringert sich beispielsweise bei Verwendung eines inerten Zwischensieders das für die Edukte zur Verfügung stehende Reaktorvolumen, d.h. die erzielbaren Raum-Zeit-Ausbeuten werden zwangsläufig kleiner. Außerdem führt beispielsweise die Mitverwendung eines reaktiven Zwischensieders, wie 2-Methyl-3-buten-2-ol, zu einer zwangsweisen Koppelung der Produktion von verschiedenen ungesättigten Ketonen, die unerwünscht sein kann.

Es war daher die Aufgabe der Erfindung, die Umsetzung von höher siedenden α,β-ungesättigten Alkoholen mit Acetessigsäurealkylestern in einer Carroll-Reaktion zu ungesättigten Ketonen so zu verbessern, daß man sie auch ohne Mitverwendung eines Lösungsmittels bzw. ohne Mitverwendung eines sogenannten Zwischensieders und damit ohne eine Koppelung mit der Herstellung anderer ungesättigter Ketone durchführen kann. Dabei sollte im Vergleich zu den in der Literatur beschriebenen Synthesen zur separaten Herstellung der ungesättigten Ketone eine höhere Produktausbeute, bezogen auf den ungesättigten Alkohol und bezogen auf den Acetessigsäurealkylester bei kürzeren Reaktionszeiten erzielt werden. Insbesondere sollten die als Zwischenprodukte für die Herstellung von dem essentiellen Vitamin-E-Vorprodukt Isophytol begehrten Ketone wie 6,10-Dimethyl-5,9-undecadien-2-on (Geranylaceton), 6,10,14-Trimethyl-5,9,13-pentadecatrien-2-on (Farnesylaceton), 6,10-Dimethyl-5-undecen-2-on (Dihydrogeranylaceton) und 6,10,14-Trimethyl-5,9-pentadecadien-2-on (Dihydrofarnesylaceton) mit höherer Selektivität und höherer Raumzeitausbeute hergestellt werden können.

Gegenstand der Erfindung ist ein Verfahren zur Herstellung von ungesättigten Ketonen der allgemeinen Formel I 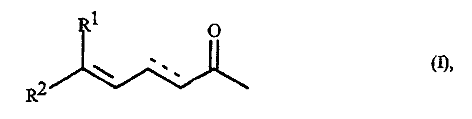 in der die gestrichelte Linie eine zusätzliche C-C-Bindung bedeuten kann, R¹ für eine Alkylgruppe mit 1 bis 4 C-Atomen steht und R² für einen gesättigten oder ungesättigten aliphatischen, cycloaliphatischen oder cycloaliphatisch-aliphatischen Rest mit 4 bis 30 C-Atomen steht, durch Umsetzen der entsprechenden α,β-ungesättigten Alkohole der allgemeinen Formel II 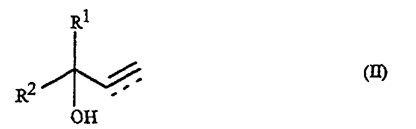 mit Acetessigsäurealkylestern der allgemeinen Formel III 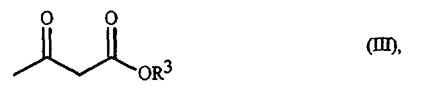 in der R³ für Alkyl mit 1 bis 4 C-Atomen steht, in Gegenwart von 0.1 bis 5 Mol-%, bezogen auf den umzusetzenden Acetessigsäurealkylester, an einer organischen Aluminiumverbindung als Katalysator unter Abspaltung und fortlaufender destillativer Entfernung von dem sich während der Reaktion bildenden Kohlendioxid und dem sich aus dem Acetessigsäureester abgespaltenen Alkohol der allgemeinen Formel IV

R³-OH (IV)

in einem Reaktorsystem mit aufgesetzter Fraktionierkolonne, das dadurch gekennzeichnet ist, daß man
- A: den α,β-ungesättigten Alkohol mit weniger als 10 Gew.-% eines inerten Lösungsmittels und mit weniger als 0,5 Gew.-% einer Flüssigkeit, die einen Siedepunkt zwischen dem des eingesetzten Acetessigsäurealkylesters der Formel III und dem des hieraus abzuspaltenden Alkohols der Formel IV aufweist, zusammen mit der organischen Aluminiumverbindung im Reaktionsgefäß vorlegt und zu diesem Gemisch den Acetessigsäurealkylester zudosiert,
- B: eine möglichst konstante Reaktionstemperatur zwischen 175°C und 220°C, vorzugsweise zwischen 180°C und 200°C, einstellt und
- C: während der Umsetzung den Gehalt an dem Acetessigsäurealkylester im Reaktionsgemisch auf einen möglichst konstanten Wert zwischen 0.1 und 10 Gew.-%, vorzugsweise zwischen 1 und 3 Gew.-%, einstellt.

Mit besonderem Vorteil gestaltet sich das Verfahren, wenn man die Einsatzmengen der Reaktanten so wählt, daß sich ein Molverhältnis von Alkohol zu Acetessigsäurealkylester zwischen 0,8 und 1,2, vorzugsweise zwischen 0,95 und 1,05, ergibt.

Mit dem beschriebenen Verfahren ergeben sich Produktausbeuten von ca. 95 % der Theorie, bezogen auf den eingesetzten Alkohol. Die Selektivität der Umsetzung, d.h. die Ausbeute, bezogen auf den umgesetzten Alkohol, liegt sogar bei über 97 % der Theorie, so daß bei einer möglichen Rückführung des nicht umgesetzten Alkohols Gesamtausbeuten von fast 100 % erreicht werden können. Die Produktausbeuten bezüglich des Acetessigsäurealkylesters liegen zwischen 90 % und 95 % bei vollständigem Abbau dieses Reaktanten. Ist ein vollständiger Abbau des Acetessigsäurealkylesters nicht erforderlich, so kann die Selektivität bezogen auf den eingesetzten Alkohol um bis zu 2 Prozentpunkte erhöht werden, wenn man den Acetessigsäureester im Überschuß einsetzt (Molverhältnis Alkohol zu Acetessigsäureester zwischen 0.7 und 0.9). In diesem Fall ist jedoch mit Einbußen bei der Selektivität bezogen auf den Acetessigester zu rechnen, so daß sich diese Vorgehensweise nur bei hochpreisigen Alkoholen lohnt. Eine Rückführung der nicht umgesetzten Reaktanten ist in jedem Fall sinnvoll.

Es war sehr überraschend, daß bei Anwendung der erfindungsgemäßen Bedingungen, d.h. insbesondere trotz Verzicht auf die Mitverwendung eines Lösungsmittels bzw. eines Zwischensieders und Anwendung von Reaktionstemperaturen von 175 bis 220, vorzugsweise 180 bis 200°C, kaum Nebenreaktionen auftreten und dadurch ausgezeichnete Selektivitäten und außerdem hohe Raum-Zeit-Ausbeuten an den ungesättigten Ketonen erzielt werden konnten. Dies gilt besonders, da in der Beschreibung von DE-A-29 28 944 anhand von vergleichsversuchen gezeigt wurde, daß ohne Zusatz von sogenannten Zwischensiedern bei den dort üblichen Reaktionsbedingungen wesentlich schlechtere Selektivitäten erzielt wurden.

Das erfindungsgemäße Verfahren läßt sich prinzipiell auf alle bekannten Varianten der sogenannten Carroll'schen Reaktion anwenden, bei denen der eingesetzte ungesättigte Alkohol höher siedet als der verwendete Acetessigsäurealkylester. Besondere Bedeutung besitzt das Verfahren jedoch für die Herstellung von ungesättigten Ketonen,die zur Herstellung von Isophytol, einem essentiellen Vorprodukt von Vitamin E, benötigt werden, d.h. im wesentlichen α,β-ungesättigte Ketone, die gebildet werden, wenn wenn man als ungesättigten Alkohol einen Alkohol der allgemeinen Formel II verwendet, in der R¹ für eine Alkylgruppe mit 1 bis 4 C-Atomen, insbesondere eine Methylgruppe steht, und R² für eine Gruppe der allgemeinen Formel VI 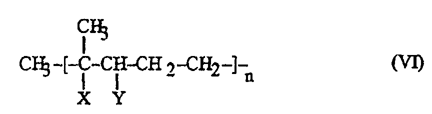 steht, in der n für eine ganze Zahl von 1 bis 6 steht und x und y entweder beide für H stehen, oder x für Methoxy und y für H steht, oder aber x und y zusammen eine zusätzliche Bindung zwischen den x- und y-tragenden C-Atomen bedeuten. Besondere Bedeutung hat das Verfahren, wenn man als Alkohol der allgemeinen Formel II beispielsweise 3,7-Dimethyl-1,6-octadien-3-ol (Linalool), 3,7-Dimethyl-1-octen-3-ol (Dihydrolinalool), 3,7,11-Trimethyl-1,6,10-dodecatrien-3-ol (Nerolidol), 3,7,11-Trimethyl-1-dodecen-3-ol oder 3,7,11-Trimethyl-1,6-dodecadien-3-ol (Dihydronerolidol) verwendet.

Die Reaktion gelingt prinzipiell mit beliebigen Acetessigsäurealkylestern, jedoch werden der Methylester, der Ethylester und der Isopropylester sowohl aus wirtschaftlichen als auch aus verfahrenstechnischen Gründen bevorzugt, da die hieraus abzuspaltenden Alkohole besonders niedrig sieden und so leicht aus dem Reaktionsgemisch entfernt werden können. Genannt werden soll aber auch der Acetessigsäure-tert.-butylester, da mit diesem die Umsetzung besonders schnell und nebenproduktfrei vonstatten geht.

Als organische Aluminiumverbindungen kommen für das erfindungsgemäße Verfahren Verbindungen der allgemeinen Formel 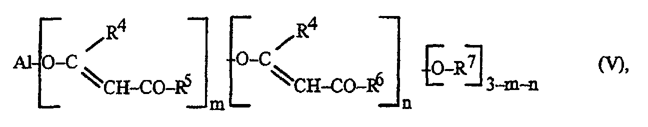 in der R⁴ Alkyl- oder Alkoxygruppen mit 1 bis 4 C-Atomen, vorzugsweise Methyl- oder Ethylgruppen bedeutet, R⁵ und R⁶ Alkyl- oder Alkoxygruppen mit 1 bis 5 C-Atomen, vorzugsweise eine Methyloder eine 2-Butylgruppe bedeuten, R⁷ für eine Alkylgruppe mit 1 bis 4 C-Atomen steht und m sowie n eine ganze Zahl von 0 bis 3 annehmen können, wobei n+m ≤ 3 ist, oder auch Aluminiumtriaryloxylate in Betracht.

Ganz besonders bevorzugt sind flüssige Aluminiumverbindungen, bei denen R⁵ ein Methylrest und R⁶ ein Butylrest, die Summe n+m = 3 und das Verhältnis n/m > 0.3 ist.

Bei den erstgenannten Katalysatoren handelt es sich also um niedere Aluminiumtrialkoholate wie Aluminiumtrimethylat, -triethylat, -triisopropylat, -tri-sek.-butylat sowie um Verbindungen, die bei der Umsetzung der genannten Aluminiumtrialkoholate mit stöchiometrischen Mengen von Acetylacetonat, Alkylacetoacetat oder Alkylmalonat unter Alkoholabspaltung und Umesterung gebildet werden. Genannt seien beispielsweise Aluminium-triacetoacetat, Aluminium-triacetylacetonat, Aluminium-monoacetoacetat-diethylat, Aluminium-monoacetoacetat-diisopropylat, Aluminium-diacetoacetat-monoisopropylat. Bevorzugt verwendet man die Aluminiumtrialkoholate, insbesondere das Aluminiumtriisopropylat sowie das Aluminium-tri-sek.-butylat. Ganz besonders bevorzugt verwendet man das gemischte Aluminium-triacetoacetat, das man durch Umsetzung von Aluminium-sek.butylat mit Acetessigsäuremethylester unter Abspaltung von 2-Butanol und Umesterung der Methoxygruppen mit dem freigesetzten 2-Butanol erhält, wobei der Umesterungsgrad über 30 % beträgt. Als Aluminiumtriaryloxylate bezeichnen wir die Aluminiumsalze von aromatischen Hydroxyverbindungen wie Aluminiumtriphenolat, Aluminiumtrikresolate, Aluminiumtrixylenolate, Aluminiumtrinaphtholate, deren Arylreste auch durch niedere Alkyl- oder Alkyloxygruppen, d.h. Alkyl- oder Alkyloxygruppen mit 1 bis 4 C-Atomen , Hydroxygruppen oder Phenyl substituiert sein können. Mit besonderem Vorteil verwendet man das relativ leicht zugängliche Aluminiumtriphenolat.

Die Menge der Aluminiumverbindung wird allgemein so bemessen, daß ihre Konzentration im Reaktionsgemisch 0.05 Gew.-% Al nicht unterschreitet und zu Beginn der Reaktion 6 Gew.-% Al nicht überschreitet. Bezogen auf umzusetzenden Acetessigsäurealkylester werden im allgemeinen 0.5 bis 5 Mol-% an der Aluminiumverbindung benötigt. Für das bevorzugt verwendete Aluminiumtriisopropylat und das oben beschriebene gemischte, aus Aluminium-sek.-butylat und Acetessigsäuremethylester hergestellte Aluminiumtriacetoacetat werden z.B. Mengen von etwa 1 bis 3 Mol-%, bezogen auf den umzusetzenden Acetessigsäurealkylester eingesetzt.

### Zu A

Unter dem Begriff inerte Lösungsmittel im Sinne von Merkmal A unseres Patentanspruchs verstehen wir übliche inerte Lösungsmittel, wie sie in Carroll-Reaktionen eingesetzt werden. Flüssigkeiten mit einem Siedepunkt zwischen dem des eingesetzten Acetessigsäurealkylesters der allgemeinen Formel III und dem des hieraus abzuspaltenden Alkohols der allgemeinen Formel IV werden in DE 29 28 944 auch als "Zwischensieder" bezeichnet. Unter der Bezeichnung "wirksame Mengen eines Lösungsmittels verstehen wir erfindungsgemäß für die üblichen Lösungsmittel weniger als etwa 10 Gew.-%, vorzugsweise weniger als 3 Gew.-%, jedoch für die in geringeren Mengen eingesetzten und als "Zwischensieder" bezeichneten Flüssigkeiten weniger als 0,5 Gew.-%.

### Zu B

Eine möglichst konstante Reaktionstemperatur zwischen 175 und 220°C, vorzugsweise 180 und 200°C kann im erfindungsgemäßen Verfahren entweder durch Variation des Wärmeeintrags und/oder ggf. des Drucks und/oder durch Variation der Zugabegeschwindigkeit des Acetessigsäurealkylesters eingestellt werden. Der übliche Wärmeeintrag beträgt zwischen 1 und 100 kW/m³ Reaktionslösung, vorzugsweise zwischen 5 und 50 kW/m³. Der Druck sollte zwischen 0.1 und 6 bar betragen, vorzugsweise zwischen 0.5 und 2 bar.

Zusätzlich bzw. alternativ zu einer Variation des Wärmeeintrags und/oder des Drucks kann auch die gezielte Zufuhr eines ggf. erhitzten Inertgases zum Ausstrippen des Alkohols und somit zur gewünschten Beeinflussung der Reaktionstemperatur genutzt werden. Insbesondere kommt dazu eine teilweise oder vollständige Rückführung des bei der Carroll-Reaktion freigesetzten Kohlendioxids in Frage. Die zudosierte Inertgasmenge sollte dazu zwischen 1 und 100 Nm³ Gas pro h und m³ Reaktorvolumen betragen, vorzugsweise zwischen 5 und 25 Nm³/(h*m³).

Auch eine Variation der dosierten Menge an Acetessigsäurealkylester über die Zeit zur Erzielung einer konstanten Temperatur in der Reaktionslösung ist möglich und sinnvoll.

Für die Durchführung des erfindungsgemäßen Verfahrens ist es weiterhin von Vorteil für eine ausreichende Durchmischung des Reaktionsgemisches im Reaktionsgefäß zu sorgen. Dies kann man beispielsweise mit Hilfe eines Rührers, durch Umpumpen der Reaktionsgemisches durch einen äußeren Flüssigkeitskreislauf, durch Eintragen des Acetessigsäurealkylesters mittels einer Mischdüse und/oder aber durch Einleiten eines Inertgasstromes erreichen.

Zur Verbesserung der Durchmischung des Reaktionsgemisches sowie zur Erleichterung der destillativen Abtrennung des bei der Reaktion gebildeten Alkohols der allgemeinen Formel IV ist es auch empfehlenswert, daß man ein Inertgas in das Reaktionsgefäß einleitet und/oder das bei der Reaktion gebildete Kohlendioxid in das Reaktionsgefäß zurückzuführt. Außerdem kann zur Intensivierung der Durchmischung eine Mischdüse verwendet werden. Arbeitet man unter Umpumpen des Reaktionsgemisches durch einen äußeren Flüssigkreislaufes, dann sollte das Reaktionsgemisch durch den äußeren Flüssigkeitskreislauf zwischen 1 und 100 mal pro Stunde, vorzugsweise zwischen 5 und 20 mal pro Stunde ausgetauscht werden.

Der Acetessigester sollte zwecks guter Durchmischung direkt in den Flüssigkeitskreislauf eindosiert werden. Falls ein zusätzlicher Wärmeeintrag erwünscht ist, kann zusätzlich zur Beheizung der Reaktionsblase ein Wärmetauscher im Flüssigkeitskreislauf vorgesehen werden.

Das erfindungsgemäße Verfahren kann sowohl diskontinuierlich als auch kontinuierlich durchgeführt werden.

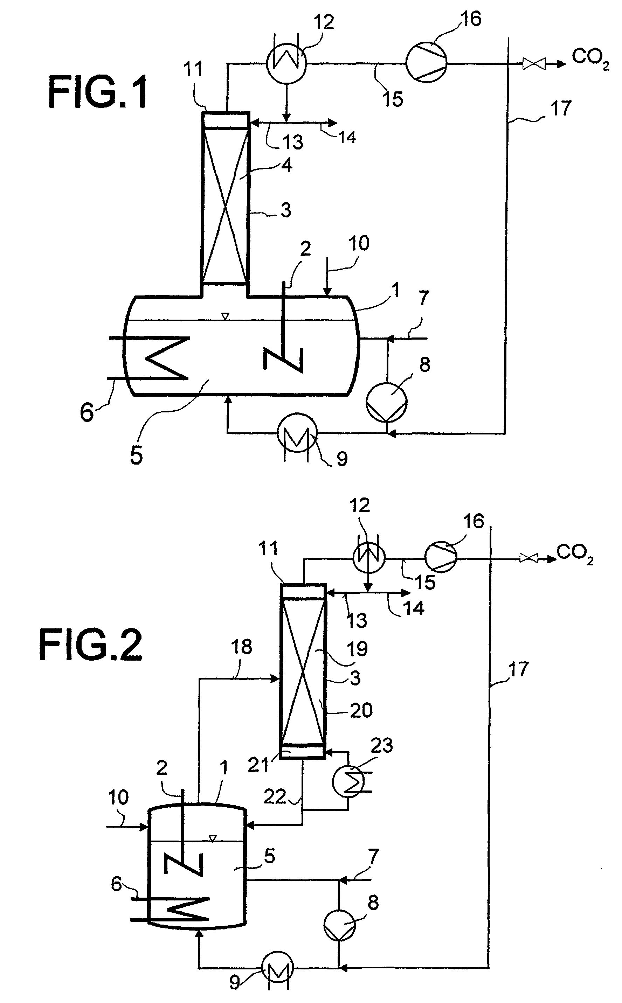

### Diskontinuierliche Durchführung des erfindungsgemäßen Verfahrens (vgl. Figur 1).

Als Reaktorsystem für die diskontinuierliche Reaktionsführung eignet sich beispielsweise die in Figur 1 dargestellte übliche beheizte Reaktionsblase (1) mit Rührer (2) und aufgesetzter Fraktionierkolonne (3), insbesondere mit einer Kolonne mit etwa 2 bis 40 theoretischen Stufen. Dabei sind alle Typen von Kolonneneinbauten (4) wie Packungen mit geordneter Struktur, Böden verschiedener Bauart oder regellose Schüttungen von Kolonnenfüllkörpern denkbar. Die Temperatur im Reaktionssystem kann durch eine Variation des Wärmeeintrages, die Änderung des Druckes, durch Zufuhr von erwärmtem oder gekühltem Inertgas und/oder durch eine geeignete variable Dosierung des Acetessigsäurealkylesters auf dem gewünschten Niveau gehalten werden.

Erfindungsgemäß wird der ungesättigte Alkohol der Formel II, gegebenenfalls zusammen mit der als Katalysator verwendeten organischen Aluminiumverbindung, in der Reaktionsblase (1) vorgelegt. Es ist von Vorteil, zusätzlich ca. 5 Gew.-% des Alkohols der allgemeinen Formel IV vorzulegen, um die Gefahr eines Siedeverzuges zu vermeiden. Der Blaseninhalt (5) wird beispielsweise mit einem Heizregister als Wärmequelle (6) oder mit einem im äußeren Kreislauf (7) mit Pumpe (8) installierten Wärmetauscher (9) aufgeheizt. Nach Einstellen der gewünschten Reaktionstemperatur wird der Acetessigsäurealkylester der Formel III über einen Zulauf (10) so in die Reaktionsblase (1) oder in den äußeren Kreislauf (7) zudosiert, daß ein möglichst konstanter Gehalt an Acetessigsäurealkylesters zwischen 0.5 und 10 Gew.-%, vorzugsweise zwischen 1 und 3 Gew.-%, in der Reaktionslösung (5) erreicht wird.

Mit dem Beginn der Dosierung des Acetessigsäurealkylesters setzt die Bildung von Alkohol der allgemeinen Formel R³-OH und Kohlendioxid ein. Diese Reaktionsprodukte werden mit Hilfe der aufgesetzten Kolonne (3) über den Kopf (11) der Kolonne abgetrennt und in den Kondensator (12) geleitet. Der auskondensierte Alkohol R³-OH wird zum einen Teil als Rücklauf (13) zurück auf die Kolonne (3) gegeben und zum anderen Teil am Auslaß (14) als Destillat abgezogen. Das den Kondensator verlassende Kohlendioxid (15) kann ganz oder teilweise mit Hilfe eines Kompressors (16) aus den oben genannten Gründen über die Gasleitung (17) in die Reaktionsblase (1) zurückgeführt werden. Die Dosierung des Acetessigsäurealkylesters über den Zulauf (10) dauert im allgemeinen etwa 2 bis 4 Stunden (h). Wenn quantitativer Umsatz des Acetessigsäurealkylesters erwünscht ist, ist es vorteilhaft, das Reaktionsgemisch nach Beendigung des Zulaufs noch etwa 1 bis 2 h bei der Reaktionstemperatur zu halten. Das Fortschreiten der Reaktion kann anhand der Entwicklung des Kohlendioxids (15) und/oder anhand der Menge des vom Acetessigsäurealkylester abgespaltenen Alkohols (14) verfolgt werden. Die Konzentration des Acetessigsäurealkylesters im Reaktionsgemisch (5) kann durch gaschromatografische Analyse bestimmt werden.

### Alternative diskontinuierliche Verfahrensführung gemäß Figur 2:

Als Variante der oben beschriebenen Verfahrensführung ist auch die in Fig.2 dargestellte Durchführung der Umsetzung in einer Reaktionsblase (1) mit einer nicht direkt aufgesetzten Fraktionierkolonne (3) möglich. Auch hierbei ist die Reaktionsblase (1) gegebenenfalls mit einem Rührer (2) und einem Heizregister als Wärmequelle (6) und ggf. mit einem äußeren Kreislauf (7) enthaltend eine Pumpe (8) und ggf. eine Wärmequelle (9) ausgestattet. Die Zudosierung des Acetessigsäurealkylesters erfolgt durch den Zulauf (10). Die aus der Reaktionsblase (1) kommenden Brüden (18) werden in eine Kolonne (3) mit Verstärkungsteil (19) und Abtriebsteil (20) geleitet. Dabei wird das entstehende Kohlendioxid zusammen mit dem entstehenden Alkohol über den Kopf der Kolonne (11) abgetrennt und in den Kondensator (12) geleitet. Analog zur oben beschriebenen Variante wird der Alkohol R³OH auskondensiert und zum Teil als Rücklauf (13) auf die Kolonne gegeben, zum anderen Teil am Auslaß (14) entnommen. Ebenso kann das den Kondensator (12) verlassende Kohlendioxid (15) mit einem Kompressor (16) über die Leitung (17) in die Reaktionsblase (1) zurückgeführt oder aus dem Reaktionskreislauf entfernt werden. Der nicht umgesetzte Reaktanten enthaltende Ablauf (22) am Sumpf (21) der Kolonne (3) wird in die Reaktionsblase (1) zurückgeführt und/oder nach Erhitzen in einer Wärmequelle (23) in den unteren Teil der Kolonne zurückgeführt. Die Dosierung und Reaktionsführung entsprechen dem oben beschriebenen Verfahren.

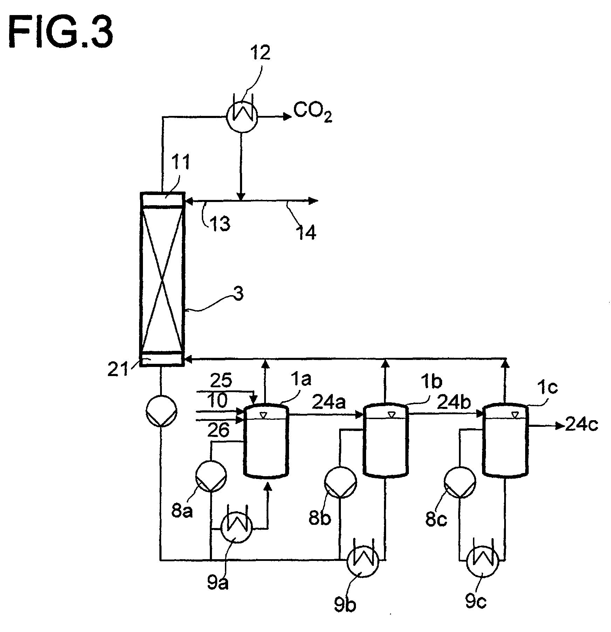

### Kontinuierliche Reaktionsführung (vgl. Figur 3):

Bei einer kontinuierlichen Verfahrensführung kann als Reaktorsystem beispielsweise eine beheizte Kesselkaskade mit 1 bis 10, zweckmäßigerweise mit 2 bis 4 Kesseln, verwendet werden. Hierbei sind die einzelnen Kessel (z.B. 1a-1c) durch die Überläufe (z.B. 24a-24c) miteinander verbunden. Es kann jedem Kessel eine separate Kolonne aufgesetzt sein oder aber - wie in Figur 3 dargestellt - nur eine Kolonne (3) für alle Kessel. Die Reaktanten werden kontinuierlich in den ersten Kessel (1a) eingebracht und zwar die Alkohole der allgemeinen Formel II über den Zulauf (25), der Acetessigsäurealkylester über den Zulauf (10) sowie gegebenenfalls der Aluminiumkatalysator über den Zulauf (26). Für das Einhalten der gewünschten Reaktionstemperatur und die Arbeitsweise der Kolonne (3) gilt im wesentlichen das gleiche wie für die diskontinuierliche Fahrweise.

Mit Hilfe des erfindungsgemäß verbesserten Verfahrens ist es möglich, zahlreiche höhere Ketone, insbesondere solche Ketone, wie sie zur Herstellung von Isophytol und damit zur Herstellung von Vitamin E benötigt werden, wie das Geranylaceton, das Farnesylaceton, 6,10-Dimethyl-5-undecen-2-on und 6,10,14-Trimethyl-5,10-pentadecadien-2-on, bei nahezu quantitativem Umsatz in sehr hohen Ausbeuten und Raumzeitausbeuten sowie hoher Reinheit herzustellen.

### Beispiel 1

### A Herstellung von 6,10-Dimethyl-5-undecen-2-on (Dihydrogeranylaceton: H-GAC) ausgehend von 3,7-Dimethyl-l-octen-3-ol (H-LIN) ohne Inertgaseinspeisung

Die Versuchsapparatur bestand aus einem elektrisch beheizbaren, mit Rührer ausgestatteten 4-Liter-Rundkolben aus Glas, auf den eine Destillationskolonne mit einem Innendurchmesser von 30 mm aufgesetzt war. Der Rundkolben war zur Dosierung der Reaktanten und zur Probenentnahme mit entsprechenden Stutzen versehen. Die Kolonne war mit einer 1 Meter hohen Schüttung aus 5-mm-Edelstahldrahtwendeln gefüllt. Die Reaktanten wurden entweder in der Reaktionsblase vorgelegt oder mit Hilfe einer Pumpe aus einem Vorlagebehälter massenstromgeregelt zudosiert. Die während der Reaktion freigesetzten Verbindungen Methanol und CO₂ wurden über die Kolonne abgetrennt und in einem Teilkondensator kondensiert. Das Kondensat lief über einen Rückflußteiler in einen Vorlagebehälter. Der verbleibende Abgasstrom wurde durch eine Kühlfalle und anschließend zur Volumenmessung durch eine Gasuhr geleitet. Die Laborapparatur war vollautomatisiert, so daß alle ein- und austretenden Stoffströme während des gesamten Versuchs kontinuierlich erfaßt und registriert werden konnten.

31.0 g Aluminiumtriisopropylat (1.54 Mol-% bezogen auf die Gesamtmenge an Acetessigsäuremethylester) wurden in einem 500 ml Rührkolben mit 77.3 g H-LIN (94%ig; 0.47 mol berechnet auf 100 %) und 17.6 g (0.15 mol) Acetessigsäuremethylester (AME) vermischt und auf 150°C aufgeheizt.

Danach wurden 1513.7 g H-LIN (94%ig; 9.12 mol berechnet auf 100 %) in dem Reaktionskolben mit aufgesetzter Kolonne vorgelegt und auf 100°C aufgeheizt. Die 150°C heiße Katalysatorlösung wurde dann mit dem 100°C heißen H-LIN in der Reaktionsblase vermischt und die erhaltene Mischung anschließend auf 175°C aufgeheizt. Hierzu wurden innerhalb von 3,5 Stunden (h) 1116.4 g (9.62 mol) AME dosiert. Während der AME-Zugabe wurde die Reaktionslösung weiter aufgeheizt und die Temperatur bei 185°C eingeregelt. Mit Beginn der AME-Zugabe setzte die CO₂-Entwicklung ein, und das gebildete Methanol wurde am Kopf der Kolonne als Destillat abgenommen. Mit Abschluß der AME-Dosierung begann die Nachreaktion. Auch während der Nachreaktion, die nach 1h beendet war, wurde die Temperatur konstant bei 185°C gehalten. Das 6,10-Dimethyl-5-undecen-2-on (H-GAC) wurde mit einer Ausbeute von 93.0 % der Theorie (d.Th.), bezogen auf eingesetztes H-LIN und 91.1 % d.Th., bezogen auf AME erhalten. Die Selektivität betrug 97.4 % d. Th., bezogen auf umgesetztes H-LIN und 91.1 % d. Th., bezogen auf AME (der Umsatz betrug 100 %, bezogen auf AME).

### Beispiel 2

### Herstellung von 6,10-Dimethyl-5-undecen-2-on (H-GAC) ausgehend von H-LIN unter Inertgaseinspeisung

Auf die in Beispiel 1A beschriebene Weise, jedoch mit zusätzlicher Einspeisung von Inertgas wurden die in Tabelle 1 erläuterten Versuche 2b und 2d durchgeführt. Hierbei wurde mit dem Beginn der AME-Dosierung aus einer Gasflasche CO₂ in die Reaktionsblase eindosiert und bis zum Abschluß der Nachreaktionsphase aufrechterhalten. Durch die Inertgaseinspeisung wurde einerseits die Durchmischung des Reaktionsgemisches gefördert und dadurch die Gefahr von Siedeverzügen vermieden und andererseits das entstehende Methanol ausgestrippt.

In der folgenden Tabelle 1 sind die erhaltenen Versuchsergebnisse dieser Beispiele 2b und 2d im Vergleich zu entsprechenden Versuchen ohne Inertgaseinspeisung (Beispiele 2a und 2c) dargestellt. Die Ergebnisse zeigen deutlich, daß die für einen vollständigen AME-Umsatz erforderliche Reaktionszeit durch die Inertgaseinspeisung verkürzt wird.

**Tabelle 1:**

| Vergleich von Versuchen mit und ohne Inertgaseinspeisung | | | | | |
|---|---|---|---|---|---|
| Beispiel | Katalysator [Mol-%] | Einsatz H-LIN/AME [mol/mol] | Ausbeute H-GAC [%] | Nachreaktionszeit [h] | Inertgaseinspeisung |
| 2a | 1.80 | 1.0 | 92.9 | 1.0 | Ohne |
| 2b | 1.80 | 1.0 | 92.6 | 0.0 | mit CO₂ |
| 2c | 1.35 | 1.0 | 89.8 | 1.0 | Ohne |
| 2d | 1.35 | 1.0 | 90.0 | 0.0 | mit CO₂ |

### Beispiel 3

### Herstellung von 6,10,14-Trimethyl-5,9-pentadecadien-2-on (Dihydrofarnesylaceton)

Die Versuchsapparatur bestand aus einem beheizbaren, mit Rührer ausgestatteten 2-Liter-Reaktionskolben aus Edelstahl, auf den eine Destillationskolonne (Länge: 1m, Durchmesser: 25 mm) aufgesetzt ist. Die Kolonne war mit Edelstahldrahtwendeln (5 mm) gefüllt. Die Reaktanten wurden entweder in der Reaktionsblase . vorgelegt bzw. mit Hilfe einer Pumpe zudosiert. Die während der Reaktion freigesetzten Verbindungen Methanol und CO₂ wurden über die Kolonne abgetrennt und kondensiert. Alle ein- und austretenden Stoffströme wurden während des gesamten Versuchs kontinuierlich erfaßt und registriert, so daß eine zeitabhängige Massenbilanzierung möglich war.

7.5 g Aluminiumtriisopropylat (1.25 Mol-% bezogen auf Gesamtmenge an AME wurden in einem 100 ml Rührkolben mit 35.4 g 3,7,11-Trimethyl-3-hydroxy-1,6-dodecadien (93%ig, 0.15 mol, berechnet auf 100 %) und 7.5 g (0.06 mol) AME vermischt und die Mischung auf 150°C aufgeheizt. Danach wurden 657.6 g 3,7,11-Trimethyl-3-hydroxy-1,6-dodecadien (93%ig, 2.73 mol, berechnet auf 100 %) in dem Reaktionskolben mit aufgesetzter Kolonne vorgelegt und auf 100°C aufgeheizt. Die 150°C heiße Katalysatorlösung wurde anschließend mit dem 100°C heißen 3,7,11-Trimethyl-3-hydroxy-1,6-dodecadien in der Reaktionsblase vermischt und auf 175°C aufgeheizt. Dann wurden 326.5 g (2.81 mol) AME innerhalb von 3.5 h in die Reaktionsblase dosiert. Während der AME-Zugabe wurde die Reaktionslösung weiter aufgeheizt und die Temperatur bei 185°C eingeregelt. Mit Beginn der AME-Zugabe setzte die CO₂-Entwicklung ein. Das gebildete Methanol wurde am Kopf der Kolonne als Destillat abgenommen. Das Rücklaufverhältnis betrug 0,1. Mit Abschluß der AME-Dosierung begann die Nachreaktion. Auch während der 2-stündigen Nachreaktion wurde die Temperatur konstant bei 185°C gehalten. Das 6,10,14-Trimethyl-pentadeca-5,10-dien-2-on wurde mit einer Ausbeute von 89.3 %, bezogen auf eingesetztes 3,7,11-Trimethyl-3-hydroxy-1,6-dodecadien und 89.3 %, bezogen auf AME erhalten. Die Selektivität betrug 93.8 %, bezogen auf 3,7,11-Trimethyl-3-hydroxy-1,6-dodecadien und 89.8 %, bezogen auf AME (der Umsatz betrug 100 %, bezogen auf AME).

## Patentansprüche

1. Verfahren zur Herstellung von ungesättigten Ketonen der allgemeinen Formel I 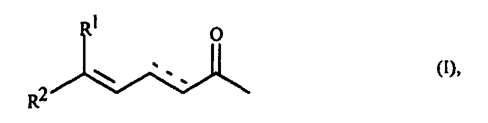 in der die gestrichelte Linie eine zusätzliche C-C-Bindung bedeuten kann, R¹ für eine Alkylgruppe mit 1 bis 4 C-Atomen steht und R² für einen gesättigten oder ungesättigten aliphatischen, cycloaliphatischen oder cycloaliphatisch-aliphatischen Rest mit 4 bis 30 C-Atomen steht, durch Umsetzen der entsprechenden α,β-ungesättigten Alkohole der allgemeinen Formel II 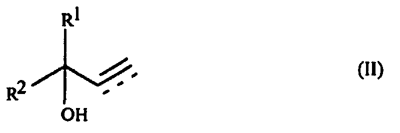 mit Acetessigsäurealkylestern der allgemeinen Formel III 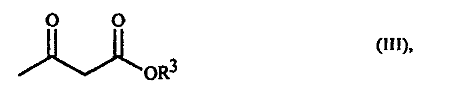 in der R³ für Alkyl mit 1 bis 4 C-Atomen steht, in Gegenwart von 0.1 bis 5 Mol-%, bezogen auf den umzusetzenden Acetessigsäurealkylester, an einer organischen Aluminiumverbindung als Katalysator unter Abspaltung und fortlaufender destillativer Entfernung von dem sich während der Reaktion bildenden Kohlendioxid und dem sich aus dem Acetessigsäureester abgespaltenen Alkohol der allgemeinen Formel IV
R³-OH (IV)
in einem Reaktorsystem mit aufgesetzter Fraktionierkolonne, **dadurch gekennzeichnet, daß** man
A den α,β-ungesättigten Alkohol mit weniger als 10 Gew.-% eines inerten Lösungsmittels und mit weniger als 0,5 Gew.-% einer Flüssigkeit, die einen Siedepunkt zwischen dem des eingesetzten Acetessigsäurealkylesters der Formel III und dem des hieraus abzuspaltenden Alkohols der Formel IV aufweist, zusammen mit der organischen Aluminiumverbindung im Reaktionsgefäß vorlegt und zu diesem Gemisch den Acetessigsäurealkylester zudosiert,
B eine möglichst konstante Reaktionstemperatur zwischen 175°C und 220°C einstellt und
C während der Umsetzung den Gehalt an dem Acetessigsäurealkylester im Reaktionsgemisch auf einen möglichst konstanten Wert zwischen 0.1 und 10 Gew.-% einstellt,

2. Verfahren gemäß Anspruch 1, **dadurch gekennzeichnet, daß** man gemäß B. eine möglichst konstante Reaktionstemperatur zwischen 180°C und 200°C einstellt.

3. Verfahren gemäß Anspruch 1, **dadurch gekennzeichnet, daß** man als organische Aluminiumverbindung eine Aluminiumverbindung der allgemeinen Formel V 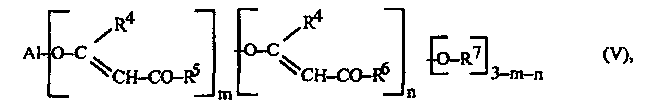 in der R⁴ eine Alkyl- oder Alkoxygruppen mit 1 bis 4 C-Atomen bedeutet, R⁵ und R⁶ Alkyl- oder Alkoxygruppen mit 1 bis 5 C-Atomen bedeuten, R⁷ für eine Alkylgruppe mit 1 bis 4 C Atomen steht und m sowie n eine ganze Zahl von 0 bis 3 bedeuten können, wobei n+m ≤ 3 ist, oder aber ein Aluminiumtriaryloxylat verwendet.

4. Verfahren gemäß Anspruch 1, **dadurch gekennzeichnet, daß** man die Einsatzmengen der Reaktanten so wählt, daß sich ein Molverhältnis von Alkohol zu Acetessigsäurealkylester zwischen 0,7 und 1,2 ergibt.

5. Verfahren gemäß Anspruch 1, **dadurch gekennzeichnet, daß** man die Einsatzmengen der Reaktanten so wählt, daß sich ein Molverhältnis von Alkohol zu Acetessigsäurealkylester zwischen 0,95 und 1,05, ergibt.

6. Verfahren gemäß Anspruch 1, **dadurch gekennzeichnet, daß** man ein Inertgas in das Reaktionsgefäß einleitet und/oder das bei Reaktion gebildete Kohlendioxid in das Reaktionsgefäß zurückführt um die Durchmischung des Reaktionsgemisches zu verbessern und die destillative Abtrennung des bei der Reaktion gebildeten Alkohols der allgemeinen Formel IV zu erleichtern.

7. Verfahren gemäß Anspruch 1, **dadurch gekennzeichnet, daß** man die unter B definierte Reaktionstemperatur durch geeignete Variation des Wärmeeintrags und/oder durch Variation der Zugabegeschwindigkeit des Acetessigsäurealkylesters regelt.

8. Verfahren gemäß Anspruch 1, **dadurch gekennzeichnet, daß** man mit Hilfe eines Rührers, durch Umpumpen des Reaktionsgemisches durch einen äußeren Flüssigkeitskreislauf, durch Eintragen des Acetessigsäurealkylesters mittels einer Mischdüse oder aber durch Einleiten eines Inertgasstromes für eine ausreichende Durchmischung des Reaktionsgemisches im Reaktionsgefäß sorgt.

9. Verfahren gemäß Anspruch 1, **dadurch gekennzeichnet, daß** man als ungesättigten Alkohol einen Alkohol der allgemeinen Formel II verwendet, in der R¹ für eine Alkylgruppe mit 1 bis 4 C-Atomen und R² für eine Gruppe der allgemeinen Formel VI 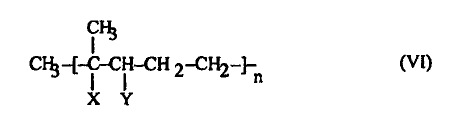 steht, in der n für eine ganze Zahl von 1 bis 6 steht und x und y entweder beide für H stehen, oder x für Methoxy und y für H stehen, oder aber x und y zusammen eine zusätzliche Bindung zwischen den x- und y-tragenden C-Atomen bedeuten.

10. Verfahren gemäß Anspruch 9, **dadurch gekennzeichnet, daß** man als Alkohol der allgemeinen Formel II 3,7-Dimethyl-1,6-octadien-3-ol (Linalool), 3,7-Dimethyl-l-octen-3-ol(Dihydrolinalool), 3,7,11-Trimethyl-1,6,10-dodecatrien-3-ol (Nerolidol), 3,7,11-Trimethyl-1-dodecen-3-ol oder 3,7,11-Trimethyl-1,6-dodecadien-3-ol (Dihydronerolidol) verwendet.

## Claims

1. A process for preparing unsaturated ketones of the general formula I 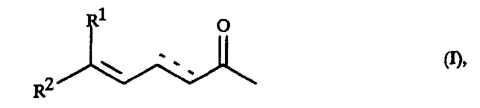 in which the dotted line may mean an additional C-C bond, R¹ is an alkyl group having 1 to 4 C atoms, and R² is a saturated or unsaturated aliphatic, cycloaliphatic or cycloaliphatic-aliphatic radical having 4 to 30 C atoms, by reacting the corresponding α,β-unsaturated alcohols of the general formula II 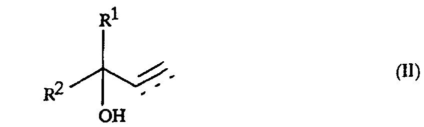 with alkyl acetoacetates of the general formula III 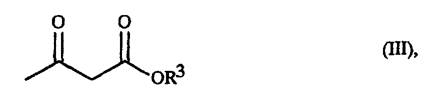 in which R³ is alkyl having 1 to 4 carbon atoms, in the presence of from 0.1 to 5 mol%, based on the alkyl acetoacetate to be reacted, of an organic aluminum compound as catalyst with elimination and continuous removal by distillation of the carbon dioxide which forms during the reaction and of the alcohol of the general formula IV
R³-OH (IV)
which is eliminated from the acetoacetate in a reactor system with fitted fractionating column, wherein
A the α,β-unsaturated alcohol is introduced with less than 10% by weight of an inert solvent and with less than 0.5% by weight of a liquid which has a boiling point between that of the alkyl acetoacetate of the formula III and that of the alcohol of the formula IV to be eliminated therefrom into the reaction vessel together with the organic aluminum compound, and the alkyl acetoacetate is metered into this mixture,
B a reaction temperature which is as constant as possible at between 175°C and 220°C is adjusted and
C during the reaction the content of alkyl acetoacetate in the reaction mixture is adjusted to a value which is as constant as possible at between 0.1 and 10% by weight.

2. A process as claimed in claim 1, wherein a reaction temperature which is as constant as possible at between 180°C and 200°C is adjusted in B.

3. A process as claimed in claim 1, wherein an aluminum compound of the general formula V 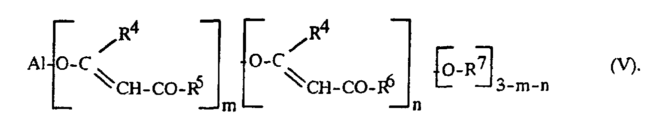 in which R⁴ is an alkyl or alkoxy group having 1 to 4 C atoms, R⁵ and R⁶ are alkyl or alkoxy groups having 1 to 5 C atoms, R⁷ is an alkyl group having 1 to 4 C atoms, and m and n may each be an integer from 0 to 3, where n+m ≤ 3, or else an aluminum triaryloxide is used as organic aluminum compound.

4. A process as claimed in claim 1, wherein the amounts of the reactants employed are chosen to result in a molar ratio of alcohol to alkyl acetoacetate of between 0.7 and 1.2.

5. A process as claimed in claim 1, wherein the amounts of the reactants employed are chosen to result in a molar ratio of alcohol to alkyl acetoacetate of between 0.95 and 1.05.

6. A process as claimed in claim 1, wherein an inert gas is passed into the reaction vessel and/or the carbon dioxide formed in the reaction is returned to the reaction vessel in order to improve the mixing of the reaction mixture and to facilitate the removal by distillation of the alcohol of the general formula IV formed in the reaction.

7. A process as claimed in claim 1, wherein the reaction temperature defined in B is controlled by suitable variation of the heat input and/or by variation of the rate of addition of the alkyl acetoacetate.

8. A process as claimed in claim 1, wherein adequate mixing of the reaction mixture in the reaction vessel is ensured by use of a stirrer, by pumping the reaction mixture through an external liquid circulation, by introducing the alkyl acetoacetate by means of a mixing nozzle or else by passing in a stream of inert gas.

9. A process as claimed in claim 1, wherein an alcohol of the general formula II in which R¹ is an alkyl group having 1 to 4 C atoms and R² is a group of the general formula VI 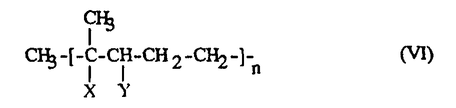 in which n is an integer from 1 to 6 and either X and Y are both H, or X is methoxy and Y is H, or else X and Y together are an additional bond between the C atoms carrying X and Y, is used as unsaturated alcohol.

10. A process as claimed in claim 9, wherein 3,7-dimethyl-1,6-octadien-3-ol (linalool), 3,7-dimethyl-1-octen-3-ol (dihydrolinalool), 3,7,11-trimethyl-1,6,10-dodecatrien-3-ol (nerolidol), 3,7,11-trimethyl-1-dodecen-3-ol or 3,7,11-trimethyl-1,6-dodecadien-3-ol (dihydronerolidol) is used as alcohol of the general formula II.

## Revendications

1. Procédé pour la préparation de cétones non saturées de formule générale I 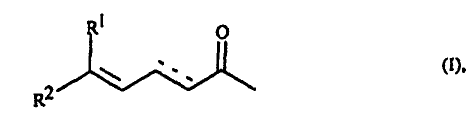 dans laquelle la ligne pointillée peut désigner une liaison C-C supplémentaire, R¹ représente un groupe alkyle ayant 1 à 4 atomes de carbone et R² désigne un reste aliphatique, cycloaliphatique ou cycloaliphatique-aliphatique, saturé ou insaturé, ayant 4 à 30 atomes de carbone, par réaction des alcools α,β-insaturés correspondants de formule générale II 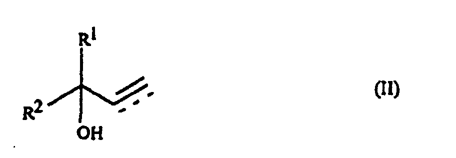 avec des esters alkyliques d'acide acétylacétique de formule générale III 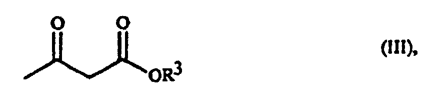 dans laquelle R³ désigne un alkyle ayant 1 à 4 atomes de carbone, en présence de 0,1 à 5 % en mol, par rapport aux esters alkyliques d'acide acétylacétique à faire réagir, d'un composé organique d'aluminium en tant que catalyseur avec séparation et élimination en continu, par distillation, du dioxyde de carbone formé pendant la réaction, et de l'alcool de formule générale IV se formant à partir de l'ester d'acide acétylacétique
R³-OH (IV)
dans un système de réacteurs comportant une colonne de fractionnement ascendante,
**caractérisé par le fait que**
A on introduit dans le réacteur, conjointement avec le composé organique d'aluminium, l'alcool α,β-insaturé avec moins de 10 % en poids d'un solvant inerte et avec moins de 0,5 % en poids d'un liquide, qui présente un point d'ébullition se situant entre celui de l'ester alkylique d'acide acétylacétique de formule III mis en oeuvre et celui de l'alcool de formule IV à éliminer, conjointement avec le composé organique d'aluminium et on ajoute au mélange, en quantité dosée, l'ester alkylique d'acide acétylacétique,
B on établit une température de réaction la plus constante possible entre 175°C et 220°C, et
C pendant la réaction, on ajuste la quantité d'ester alkylique d'acide acétylacétique contenue dans le mélange réactionnel à une valeur la plus constante possible entre 0,1 et 10 % en poids.

2. Procédé selon la revendication 1, **caractérisé par le fait qu'**on établit selon B une température de réaction la plus constante possible entre 180°C et 200°C.

3. Procédé selon la revendication 1, **caractérisé par le fait qu'**on utilise comme composé organique d'aluminium un composé d'aluminium de formule générale V 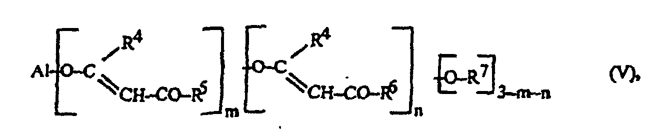 où R⁴ désigne un groupe alkyle ou alcoxy ayant 1 à 4 atomes de carbone, R⁵ et R⁶ représentent des groupes alkyle ou alcoxy ayant 1 à 5 atomes de carbone, R⁷ désigne un groupe alkyle ayant 1 à 4 atomes de carbone, et m ainsi que n peuvent représenter un nombre entier de 0 à 3, tandis que n+m ≤ 3, ou encore un triaryloxylate d'aluminium.

4. Procédé selon la revendication 1, **caractérisé par le fait qu'**on choisit la quantité introduite des réactifs de telle sorte que l'on ait un rapport molaire de l'alcool à l'ester alkylique d'acide acétylacétique entre 0,7 et 1,2.

5. Procédé selon la revendication 1, **caractérisé par le fait qu'**on choisit la quantité introduite des réactifs de telle sorte que l'on ait un rapport molaire de l'alcool à l'ester alkylique d'acide acétylacétique entre 0,95 et 1,05.

6. Procédé selon la revendication 1, **caractérisé par le fait qu'**on fait passer un gaz inerte dans le réacteur et/ou on recycle dans le réacteur le dioxyde de carbone formé dans la réaction pour améliorer le brassage du mélange réactionnel et pour faciliter l'élimination par distillation de l'alcool de formule générale IV formé au cours de la réaction.

7. Procédé selon la revendication 1, **caractérisé par le fait qu'**on règle la température de réaction définie sous B par variation appropriée de l'apport de chaleur et/ou par variation de la vitesse d'introduction de l'ester alkylique d'acide acétylacétique.

8. Procédé selon la revendication 1, **caractérisé par le fait qu'**on veille à un brassage suffisant du mélange réactionnel dans le réacteur, au moyen d'un agitateur, par recyclage par pompage du mélange réactionnel par l'intermédiaire d'un circuit externe de liquide, par introduction de l'ester alkylique d'acide acétylacétique au moyen d'un ajutage mixte ou encore par introduction d'un courant de gaz inerte.

9. Procédé selon la revendication 1, **caractérisé par le fait qu'**on utilise comme alcool insaturé un alcool de formule générale II, dans laquelle R¹ désigne un groupe alkyle ayant 1 à 4 atomes de carbone et R² représente un groupe de formule générale VI 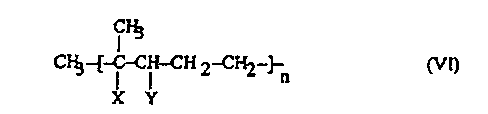 dans laquelle n désigne un nombre entier de 1 à 6, et soit x et y désignent tous deux H, soit x représente un méthoxy et y représente H, ou encore x et y désignent ensemble une liaison supplémentaire entre les atomes de carbone portant x et y.

10. Procédé selon la revendication 9, **caractérisé par le fait qu'**on utilise comme alcool de formule générale II le 3,7-diméthyl-1,6-octadiène-3-ol (linalol), le 3,7-diméthyl-1-octène-3-ol (dihydrolinalol), le 3,7,-11-triméthyl-1,6,10-dodécatriène-3-ol (nérolidol), le 3,7,11-triméthyl-1,6,10-dodécène-3-ol ou le 3,7,11-triméthyl-1,6,10-dodécadiène-3-ol (dihydronérolidol).
